# FASCICULE DE BREVET EUROPEEN

(11) **EP 3 595 593 B1**
(45) Date de publication et mention de la délivrance du brevet: **24.05.2023**
(21) Numéro de dépôt: 18708440.5
(22) Date de dépôt: 09.03.2018
(51) Int. Cl.: A61F 2/66

(54) **PROTHÈSE DE PIED COMPORTANT UN ÉLÉMENT D'AMORTISSEMENT**
FUSSPROTHESE MIT EINEM DÄMPFUNGSELEMENT
FOOT PROSTHESIS COMPRISING A DAMPING MEMBER

(30) Priorité: 17.03.2017 FR 1752217; 29.11.2017 FR 1761388
(43) Date de publication de la demande: 22.01.2020
(73) Titulaire: PM Ingenierie et Design, 91140 Villebon-sur-Yvette (FR)
(72) Inventeur: PENOT, Benjamin, 91140 Villebon-sur-Yvette (FR)
(74) Mandataire: Argyma
(86) Numéro de dépôt international: PCT/EP2018/055868
(87) Numéro de publication internationale: WO 2018/166905

(56) Documents cités:
- EP-A1- 0 280 004
- EP-A1- 0 487 852
- WO-A1-96/41598
- DE-B3-102014 006 571
- US-A1- 2005 033 450
- US-A1- 2010 023 135

## Description

La présente invention concerne d'une manière générale une prothèse de pied comportant un élément d'amortissement ayant une forme proche de celle d'un pied humain. L'invention a notamment pour objectif d'augmenter la durée de vie de ce type de prothèse et également d'améliorer le confort de marche de son utilisateur.

De nos jours, les prothèses de pied constituent une solution technique incontournable pour remplacer un pied amputé. Il est connu de l'état de la technique des prothèses de pied telles que celles décrites dans les documents WO9641598, EP0280004 ou US2749557 qui cherchent à reproduire l'anatomie d'un pied humain, mais l'inconvénient principal de ces prothèses est qu'elles ne permettent pas de reproduire la complexité des différents mouvements effectués par un pied. Par exemple, le brevet US2749557 décrit un système de cheville permettant le réglage en hauteur du talon de la prothèse ainsi que le positionnement de la pointe de pied. L'inconvénient principal de ce système est qu'il ne permet un réglage que sur un nombre limité de positions. De surcroît, cette solution de prothèse de pied ne permet ni une restitution énergétique ni une gestion de l'inversion et de l'éversion. Il existe également dans l'état de la technique des prothèses de pied comprenant une lame ressort, en matériau composite du type fibres de carbone, de verre ou d'aramide, permettant de restituer l'énergie emmagasinée lors de la phase d'appui de la prothèse sur le sol. Ce type de prothèse cherche à reproduire les mouvements d'un pied humain lors des phases d'appui ou de relance du corps. Une telle prothèse est décrite, par exemple, dans la demande de brevet WO20111066354. L'inconvénient majeur de ces prothèses est qu'il faut ajouter sur la lame ressort divers éléments permettant de reproduire l'esthétisme et l'anatomie d'un pied humain. Par ailleurs, il existe également des prothèses de pied mettant en oeuvre une simple plaque d'appui. Il est alors utilisé un système actif, appelé prothèse électronique, équipé de capteurs, permettant notamment de détecter l'appui sur le sol, et comprenant des actionneurs sur la cheville et le genou permettant de retendre le pied. Ce type de système est décrit, par exemple, dans la demande de brevet US 2012/0191220.

Une autre prothèse de pied connue est décrite dans le document EP 0487852 A1, qui divulgue un pied prothétique sans articulation, comportant un talon, une pointe, et un élément d'amortissement.

Les diverses prothèses connues actuellement ne permettent pas de reproduire de manière satisfaisante la cinématique d'un pied humain. En effet, la marche mobilise certains groupes musculaires lors de la phase d'appui, permettant l'amortissement du choc du pas, et d'autres groupes musculaires lors de la phase de propulsion, permettant la restitution énergétique. Les prothèses à lame permettent une accumulation et une restitution d'énergie relativement efficaces, mais, en revanche, elles présentent l'inconvénient de ne pas pouvoir s'adapter correctement à un sol présentant un devers important ou sur lequel des protubérances sont présentes, telles que des cailloux ou des pierres. En outre, elles ont tendance à s'user rapidement du fait du frottement généré par le contact permanent de la prothèse sur le sol.

L'invention a donc pour but d'améliorer la durée de vie des prothèses de pied et d'augmenter le confort de marche de l'utilisateur.

Pour ce faire, l'invention a notamment pour objet une prothèse de pied telle que décrite dans la revendication 1.

De préférence, l'élément d'amortissement relie la pointe de pied au support de cheville.

De préférence, la prothèse de pied comprend en outre un moyen d'accumulation et de restitution d'énergie disposé entre la pointe de pied et le coup de pied.

De manière avantageuse, la prothèse de pied comprend une bielle reliant le coup de pied au talon.

De préférence encore, l'élément d'amortissement comprend un premier point de courbure et un deuxième point de courbure.

De préférence, l'élément d'amortissement comprend une extrémité reliée au support de cheville par l'extérieur de la prothèse de pied.

Selon un autre aspect de l'invention, l'élément d'amortissement comprend une extrémité reliée au support de cheville par l'intérieur de la prothèse de pied.

Avantageusement, le support de cheville comprend en outre un moyen de réglage disposé à l'extérieur ou à l'intérieur du support de cheville.

Dans un autre aspect de l'invention, un robot comporte une prothèse de pied selon l'invention.

L'invention sera mieux comprise à la lecture de la description qui suit, faite en référence aux figures annexées, dans lesquelles :
- la figure 1 est une vue en perspective d'un exemple de prothèse de pied selon l'invention configurée pour recevoir une chaussure à talon plat ;
- la figure 2 est une vue de côté de la prothèse représentée sur la figure 1 ;
- la figure 3 est une vue de côté de la prothèse représentée sur la figure 1 configurée pour recevoir une chaussure à talon haut ;
- la figure 4 est une vue de détail d'un mécanisme de la prothèse montrée sur la figure 1 ;
- la figure 5 est une vue de détail d'un autre mécanisme de la prothèse montrée sur la figure 1.
- la figure 6 représente une vue de côté d'un autre mode de réalisation de la prothèse de pied selon l'invention ;
- la figure 7 représente une vue en perspective d'un élément d'amortissement de la prothèse de pied montrée sur la figure 6.

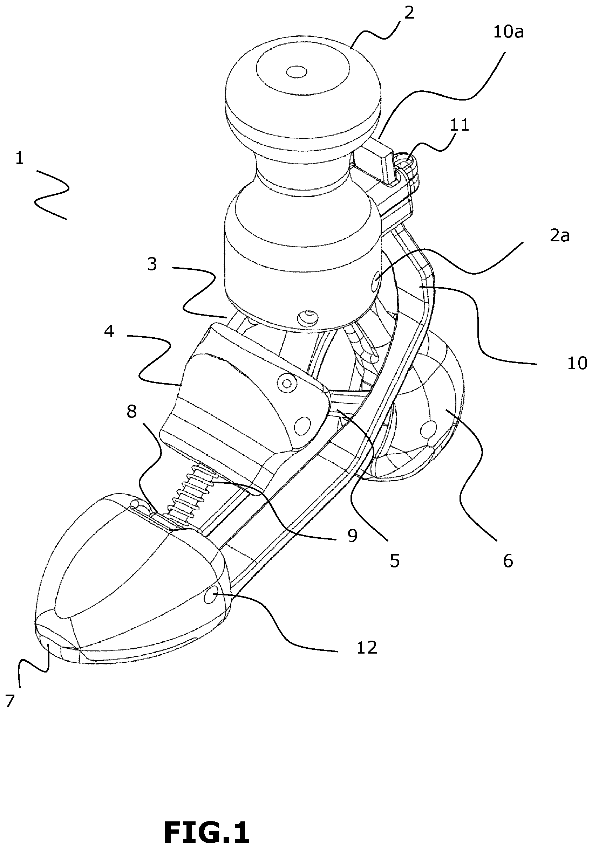

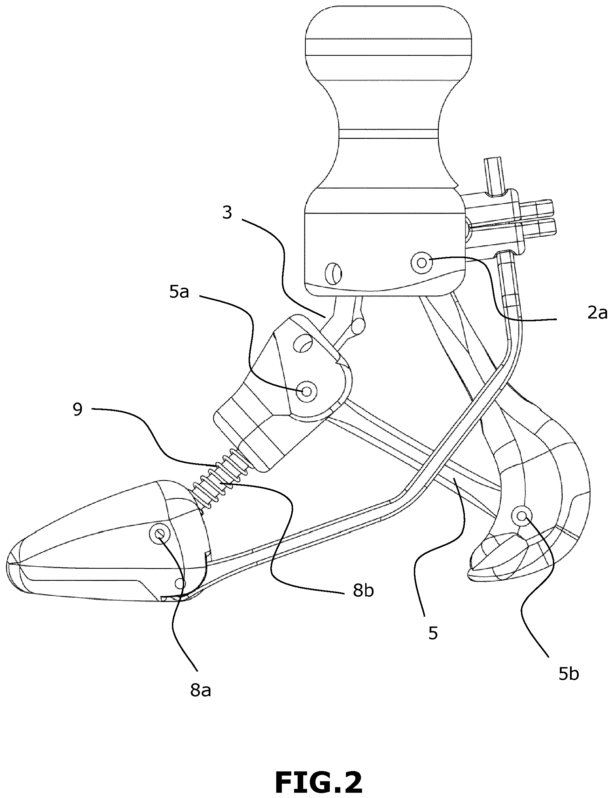

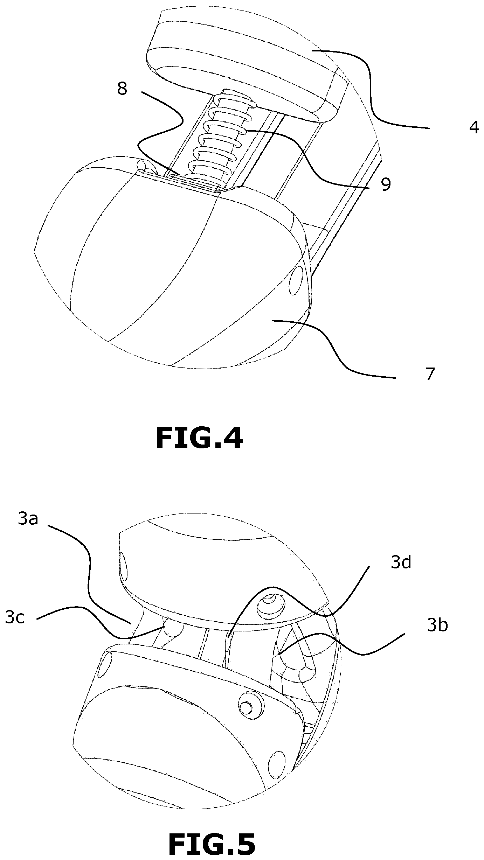

Il est représenté sur la figure 1 un exemple de mode de réalisation de la prothèse de pied 1 selon l'invention comprenant un support de cheville 2, permettant à la prothèse de pied 1 de venir s'encastrer dans le tibia du patient, lié sur sa partie inférieure à un coup de pied 4 par l'intermédiaire d'un mécanisme 3. Comme il peut être mieux vu sur la figure 5, ce mécanisme 3 comprend une première paire de biellettes 3a située à l'intérieur de la prothèse de pied 1 et une deuxième paire de biellettes 3b située à l'extérieur de la prothèse de pied 1. La première paire de biellettes 3a est articulée autour d'une liaison pivot 3c et la deuxième paire de biellettes 3b autour d'une liaison pivot 3d. On aperçoit également sur les figures 1 et 2, une biellette 5 articulée avec le coup de pied 4 au moyen de l'axe 5a, de type liaison pivot, et avec le talon 6 au moyen de l'axe 5b, également de type liaison pivot. Cette biellette 5 permet de transférer l'effort provenant du talon 6, lorsque celui-ci est posé sur le sol, vers le coup de pied 4. Le talon 6 est également articulé avec le support de cheville 2 au moyen d'un axe 2a de type liaison pivot. La prothèse de pied 1 comprend également une pointe de pied 7 liée au coup de pied 4 par l'intermédiaire d'une liaison de type cardan 8. Ce cardan 8 comprend une première articulation 8a de type liaison pivot formant un premier axe orthogonal au plan de symétrie de la pointe de pied 7 et une deuxième articulation 8b de type liaison pivot formant un deuxième axe. La deuxième articulation 8b comprend en outre un ressort 9. Dans d'autres modes de réalisation, le ressort 9 peut être remplacé par un amortisseur hydraulique ou pneumatique, ou tout autre moyen permettant d'obtenir un amortissement réglable en raideur permettant une adaptation au type de marche souhaitée par le patient telle qu'une marche sportive ou une marche citadine.

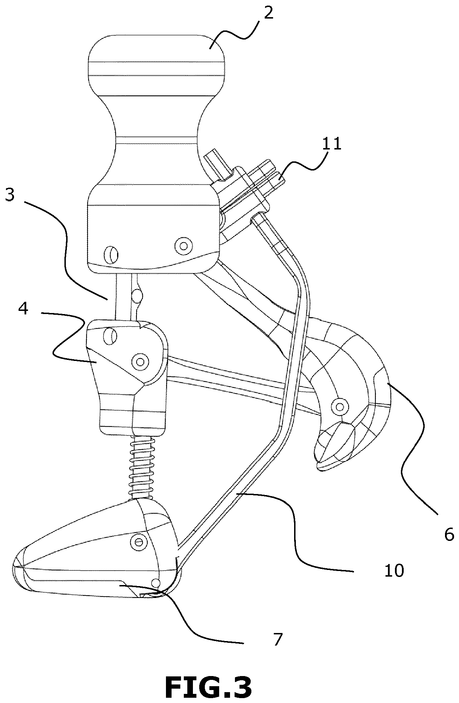

La prothèse de pied 1 comprend en outre une lame 10 en matériau composite, de type fibres de carbone, permettant un amortissement lors de la phase d'appui du talon et une accumulation d'énergie. Dans un autre mode de réalisation, la lame 10 peut être en un alliage d'aluminium, en acier à ressort, ou en tout autre matériau ayant des caractéristiques mécaniques permettant d'accumuler et de restituer de l'énergie. La lame 10 est liée à la pointe de pied 7 au moyen d'une articulation 12 de type liaison pivot et au support de cheville par l'intermédiaire d'un système de réglage 11 dans lequel l'extrémité 10a de la lame 10 vient se loger. Le système de réglage 11, comprenant un verrouillage mécanique, permet de régler les angles de dorsiflexion et de plantiflexion, l'angle d'inversion-éversion, l'angle de pointe de pied 7 par rapport à l'horizontale ainsi que la hauteur de la prothèse de pied. Ce réglage se fait en déverrouillant le système de réglage 11 et ainsi libérer temporairement la lame 10 qui peut ainsi translater de bas en haut et également se mouvoir angulairement par rapport à l'axe longitudinal de la cheville 2. Il est alors possible de régler l'angle d'inclinaison du coup de pied 4, la hauteur du talon 6, répliquant ainsi la plantiflexion, et l'angle d'inclinaison de la pointe de pied 7 par rapport au sol. Le système de réglage 11 autorise ainsi un réglage fin adapté au patient et aux critères d'usage, de confort et d'environnement. La figure 2 représente la prothèse de pied 1 réglée pour porter une chaussure à talon plat, le talon 6 étant dans une position basse. La figure 3 représente la même prothèse de pied que celle vue sur la figure 2, mais réglée pour que le patient puisse porter une chaussure à talon haut. Dans le mode de réalisation décrit sur les figures 1 à 5, il est représenté une lame 10, en forme de Y orienté vers le bas, comprenant un premier bras situé à l'extérieur de la prothèse de pied 1 et un deuxième bras situé à l'intérieur de la prothèse de pied 1. La troisième branche du Y formant la lame 10 est logée dans le système de réglage 11. Dans un autre mode de réalisation de l'invention, la lame 10 peut être constituée d'une seule lame. Dans un mode de réalisation encore différent, le système de réglage comprend un premier actionneur permettant, en temps réel, de régler la hauteur de la lame 10 par rapport au sol et un deuxième actionneur permettant de régler l'inclinaison de la lame 10 par rapport à l'horizontale. Ces actionneurs peuvent être, par exemple, des moteurs électriques ou des servomoteurs. Des capteurs d'effort sont positionnés au niveau du talon 6, de la pointe de pied 7, du ressort 9 et sur la lame 10. Les informations provenant de ces capteurs sont traitées en temps réel par un ordinateur afin d'ajuster le réglage de la prothèse de pied 1 au moyen du système de réglage 11. Dans ce mode de réalisation, il n'est pas nécessaire que le patient intervienne dans le réglage de la prothèse de pied 1 lorsque les conditions de marche changent, le réglage optimal étant effectué directement par le système de réglage 11. La lame 10 est configurée de manière à être distante du sol afin de ne pas être en contact avec ce dernier. Cela procure l'avantage d'éviter une usure par frottement de la lame 10 et ainsi augmenter fortement la durée de vie de la prothèse de pied 1.

On va maintenant décrire le fonctionnement de la prothèse de pied 1 lorsqu'un pas est effectué par le patient, ou par un robot lorsque ce dernier comporte une prothèse de pied 1. Dans un premier temps, le talon 6 vient en contact avec le sol transmettant un effort par l'intermédiaire de la biellette 5 à la lame 10 qui se déforme de manière à accumuler de l'énergie et à amortir le pas. La biellette 5 transmet également un effort, dirigé vers le talon 6, dans le coup de pied 4 qui a pour effet d'amener la pointe de pied 7 vers le sol par l'intermédiaire du cardan 8. Le ressort 9 est comprimé et accumule de l'énergie également. La prothèse de pied 1 génère ainsi un effet de stabilisation sur l'inversion-éversion, même en cas de sol présentant un devers important ou une protubérance tels qu'une descente, une montée ou un caillou.

Lorsque le patient, ou le robot, lève la prothèse de pied 1, pour effectuer le pas suivant, l'énergie accumulée dans la lame 10 et le ressort 9 est restituée afin d'aider le patient, ou le robot, à lever la prothèse de pied 1. Le patient, ou le robot, retrouve ainsi une restitution d'énergie similaire à celle d'un pied humain.

Il est à noter que les diverses pièces constituant la prothèse de pied 1 selon l'invention sont à adapter, notamment en dimensions, en fonction des paramètres du patient, ou du robot, recevant la prothèse tels que son poids, sa taille ou son type de marche.

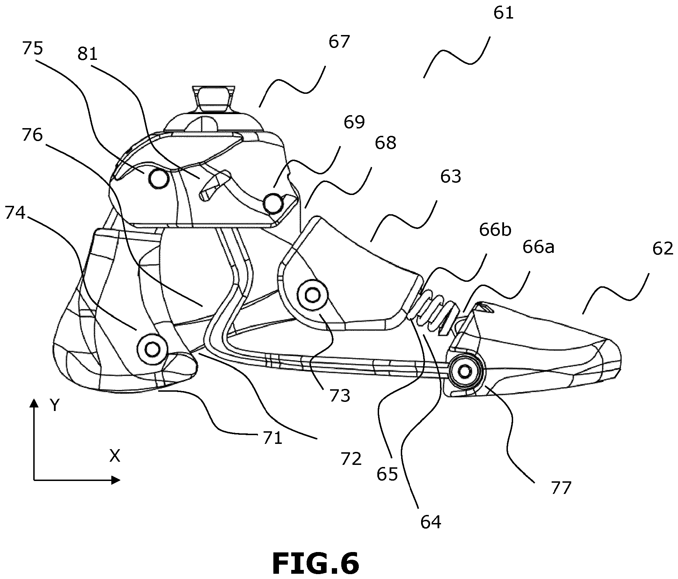

En référence à la figure 6, il est représenté de manière schématique un autre mode de réalisation d'une prothèse selon la présente invention. On peut y voir une prothèse de pied 61 comportant une pointe de pied 62 et un coup de pied 63 reliés entre eux par une liaison de type cardan 64. Le cardan 64 comprend une première articulation 66a de type liaison pivot intégrée à la pointe de pied 62 et une deuxième articulation 66b, également de type liaison pivot, intégrée au coup de pied 63. La tige de la deuxième articulation 66b comprend en outre un ressort 65. On peut voir sur la figure 6 un axe X et un axe Y, perpendiculaires entre eux. L'articulation 66a a son axe de rotation perpendiculaire au plan X-Y formé par les axes X et Y et l'articulation 66b a son axe de rotation perpendiculaire à l'axe de rotation de l'articulation 66a. Le ressort 65 a la même fonction que dans le mode de réalisation décrit sur les figures 1 à 5. Il peut également être remplacé avantageusement par un amortisseur hydraulique, pneumatique ou oléopneumatique, ou tout autre moyen permettant d'obtenir un amortissement réglable en raideur afin de s'adapter à un type de marche souhaité. En outre, le coup de pied 63 est lié à un support de cheville 67 par l'intermédiaire d'une liaison 68. La liaison 68 comporte un premier axe 69 perpendiculaire au plan X-Y formant une première liaison pivot avec le support de cheville 67 et un deuxième axe 70 (non visible) également perpendiculaire au plan X-Y, formant une deuxième liaison pivot avec le coup de pied 63. La liaison 68 est faite d'une seule pièce permettant ainsi de rigidifier la prothèse de pied 61. Le coup de pied 63 est également lié à un talon 71 par l'intermédiaire d'une bielle 72. La bielle 72 comprend une première liaison pivot 73 la reliant au coup de pied 63 et ayant son axe de rotation perpendiculaire au plan X-Y et une deuxième liaison pivot 74 la reliant au talon 71 ayant également son axe de rotation perpendiculaire au plan X-Y. Le talon 71 est articulé par rapport au support de cheville 67 au moyen d'une liaison pivot 75. La prothèse 61 comprend également un élément d'amortissement 76 liant directement la pointe de pied 62 au support de cheville 67. Cet élément d'amortissement 76 constitue une alternative à la lame 10 de la prothèse de pied 1 telle que montrée sur la figure 1. L'élément d'amortissement 76 est configuré de manière à être distant du sol afin de ne pas être en contact avec ce dernier. Cela procure l'avantage d'éviter une usure par frottement de l'élément d'amortissement 76 et ainsi augmenter fortement la durée de vie de la prothèse de pied 61.

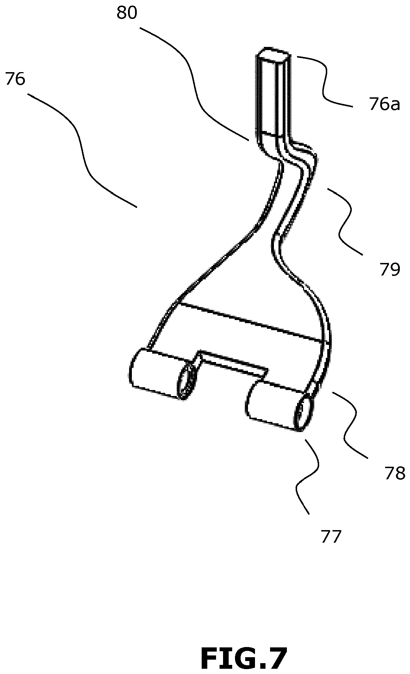

En référence à la figure 7, il est représenté schématiquement et en perspective l'élément d'amortissement 76. Il comprend une partie 78, formant un plan, liée à la pointe de pied par une liaison pivot 77. L'élément d'amortissement 76 comprend en outre un premier point de courbure 79 et un deuxième point de courbure 80. Cette forme comportant deux points de courbure a pour avantage d'améliorer l'amortissement vertical. Le matériau de l'élément d'amortissement 76 peut être métallique, par exemple de l'acier, ou un polymère ou tout autre matériau permettant un amortissement et une accumulation d'énergie lors de l'appui sur le sol de la prothèse 61 et de la marche du patient ou du robot. L'élément d'amortissement 76 comprend en outre une extrémité 76a venant se loger dans un moyen de réglage intérieur 81 de la prothèse 61. Le moyen de réglage intérieur 81 comprend un verrouillage mécanique, comme par exemple une vis de pression. Lorsque l'on désire régler la prothèse de pied 61, on déverrouille le moyen de réglage intérieur 81 pour permettre de déplacer l'élément d'amortissement 76. Dans un autre mode de réalisation, le moyen de réglage comprend un actionneur permettant de régler directement l'élément d'amortissement 76 à l'aide d'informations provenant, par exemple, d'un ordinateur. Cet actionneur peut être, par exemple, un moteur électrique ou un servomoteur.

Comme déjà mentionné, la prothèse de pied objet de l'invention peut également être mise en oeuvre dans le domaine de la robotique. En effet, il y est utilisé des pieds artificiels afin que des robots humanoïdes puissent marcher d'une manière similaire à celle d'un être humain. Ces pieds de robot sont généralement constitués d'une simple plaque d'appui pouvant être remplacée avantageusement par la prothèse de pied 1 ou la prothèse de pied 61. Une prothèse de pied selon l'invention peut également être directement incorporée au robot lors de sa fabrication.

Un des avantages de l'invention est que la lame 10 ou l'élément d'amortissement 76 ne sont pas en contact avec le sol permettant ainsi une augmentation significative de sa durée de vie par rapport aux prothèses de l'état de la technique comprenant une lame directement en contact avec le sol subissant ainsi un frottement permanent et donc une usure rapide. Un autre avantage de l'invention, par rapport aux prothèses à lame existantes, est qu'il n'est pas nécessaire d'ajouter de chaussette, par exemple en silicone, afin de protéger la prothèse des nuisances extérieures, telles que les poussières, et également pour reproduire la forme d'un pied humain.

## Revendications

1. Prothèse de pied (1, 61) comprenant un talon (6, 71) et une pointe de pied (7, 62) aptes à être en appui sur le sol et un support de cheville (2, 67), ainsi qu'un élément d'amortissement (10, 76) configuré pour être distant dudit sol, la prothèse de pied (1, 61) étant **caractérisée en ce qu'**elle comprend en outre au moins un coup de pied (4, 63) reliant ladite pointe de pied (7, 62) audit support de cheville (2, 67) et une bielle (72) reliant ledit coup de pied (4, 63) audit talon (6, 71).

2. Prothèse de pied (1, 61) selon la revendication 1 **caractérisée en ce que** l'élément d'amortissement (10, 76) relie directement la pointe de pied (7, 62) au support de cheville (2, 67).

3. Prothèse de pied (1, 61) selon l'une quelconque des revendications précédentes **caractérisée en ce qu'**elle comprend en outre un moyen d'accumulation et de restitution d'énergie disposé entre la pointe de pied (7, 62) et le coup de pied (4, 63).

4. Prothèse de pied (1, 61) selon l'une quelconque des revendications précédentes **caractérisée en ce que** l'élément d'amortissement (10, 76) comprend une extrémité (10a) reliée au support de cheville (2, 67) par l'extérieur de la prothèse de pied (1, 61).

5. Prothèse de pied (1, 61) selon l'une quelconque des revendications précédentes **caractérisée en ce que** l'élément d'amortissement (10, 76) comprend une extrémité (76a) reliée au support de cheville (2, 67) par l'intérieur de la prothèse de pied (1, 61).

6. Prothèse de pied (1, 61) selon l'une quelconque des revendications précédentes **caractérisée en ce que** le support de cheville (2, 67) comprend en outre un moyen de réglage (11, 81) disposé à l'extérieur ou à l'intérieur du support de cheville (2, 67).

7. Robot **caractérisé en ce qu'**il comporte une prothèse de pied (1, 61) selon l'une quelconque des revendications précédentes.

## Patentansprüche

1. Fußprothese (1, 61), umfassend eine Ferse (6, 71) und eine Fußspitze (7, 62), die imstande sind, in Abstützung auf dem Boden zu sein, und eine Knöchelstütze (2, 67), sowie ein Dämpfungselement (10, 76), das ausgelegt ist, um vom Boden beabstandet zu sein, wobei die Fußprothese (1, 61) **dadurch gekennzeichnet ist, dass** sie ferner mindestens einen Spann (4, 63) umfasst, der die Fußspitze (7, 62) mit der Knöchelstütze (2, 67) verbindet, und eine Stange (72), die den Spann (4, 63) mit der Ferse (6, 71) verbindet.

2. Fußprothese (1, 61) nach Anspruch 1, **dadurch gekennzeichnet, dass** das Dämpfungselement (10, 76) die Fußspitze (7, 62) direkt mit der Knöchelstütze (2, 67) verbindet.

3. Fußprothese (1, 61) nach einem der vorangehenden Ansprüche, **dadurch gekennzeichnet, dass** sie ferner ein Energieakkumlations- und Restitutionsmittel umfasst, das zwischen der Fußspitze (7, 62) und dem Spann (4, 63) angeordnet ist.

4. Fußprothese (1, 61) nach einem der vorangehenden Ansprüche, **dadurch gekennzeichnet, dass** das Dämpfungselement (10, 76) ein Ende (10a) umfasst, das mit der Knöchelstütze (2, 67) außerhalb der Fußprothese (1, 61) verbunden ist.

5. Fußprothese (1, 61) nach einem der vorangehenden Ansprüche, **dadurch gekennzeichnet, dass** das Dämpfungselement (10, 76) ein Ende (76a) umfasst, das mit der Knöchelstütze (2, 67) innerhalb der Fußprothese (1, 61) verbunden ist.

6. Fußprothese (1, 61) nach einem der vorangehenden Ansprüche, **dadurch gekennzeichnet, dass** die Knöchelstütze (2, 67) ferner ein Einstellmittel (11, 81) umfasst, das außerhalb oder innerhalb der Knöchelstütze (2, 67) angeordnet ist.

7. Roboter, **dadurch gekennzeichnet, dass** er eine Fußprothese (1, 61) nach einem der vorangehenden Ansprüche aufweist.

## Claims

1. Foot prosthesis (1, 61) comprising a heel (6, 71) and a foot tip (7, 62) capable of bearing on the ground and an ankle support (2, 67), as well as a damping element (10, 76) configured to be distant from said ground, the foot prosthesis (1, 61) being **characterised in that** it further comprises at least an instep (4, 63) connecting the foot tip (7, 62) to the ankle support (2,67), and a connecting rod (72) connecting the instep (4, 63) to the heel (6,71).

2. Foot prosthesis (1, 61) according to claim 1 **characterised in that** the damping element (10, 76) directly connects the foot tip (7, 62) to the ankle support (2, 67).

3. Foot prosthesis (1, 61) according to any of the preceding claims **characterised in that** it further comprises a means for accumulating and restoring energy arranged between the foot tip (7, 62) and the instep (4, 63).

4. Foot prosthesis (1, 61) according to any of the preceding claims **characterised in that** the damping element (10, 76) comprises one end (10a) connected to the ankle support (2, 67) by the outside of the foot prosthesis (1, 61).

5. Foot prosthesis (1, 61) according to any of the preceding claims **characterised in that** the damping element (10, 76) comprises one end (76a) connected to the ankle support (2, 67) by the inside of the foot prosthesis (1, 61).

6. Foot prosthesis (1, 61) according to any of the preceding claims **characterised in that** the ankle support (2, 67) further comprises an adjustment means (11, 81) arranged outside or inside the ankle support (2, 67).

7. Robot **characterised in that** it comprises a foot prosthesis (1, 61) according to any of the preceding claims.
